(19) Europäisches Patentamt
European Patent Office
Office européen des brevets

(11) EP 2 181 082 B1

(12) **EUROPEAN PATENT SPECIFICATION**

(45) Date of publication and mention of the grant of the patent:
**25.04.2012 Bulletin 2012/17**

(21) Application number: **08774705.1**

(22) Date of filing: **03.07.2008**

(51) Int Cl.:
***C07C 17/392*** *(2006.01)* ***C07C 17/389*** *(2006.01)*
***C07C 19/16*** *(2006.01)* ***C07C 211/63*** *(2006.01)*

(86) International application number:
**PCT/EP2008/058588**

(87) International publication number:
**WO 2009/007302 (15.01.2009 Gazette 2009/03)**

(54) **PROCESS FOR THE PURIFICATION OF ALPHA,OMEGA-DIIODOPERFLUORINATED COMPOUNDS**

VERFAHREN ZUR AUFREINIGUNG VON ALPHA, OMEGA-DIIODPERFLUORINIERTEN VERBINDUNGEN

PROCEDE DE PURIFICATION DE COMPOSES ALPHA-OMEGA-DIIODOPERFLUORES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.07.2007 IT MI20071384**

(43) Date of publication of application:
**05.05.2010 Bulletin 2010/18**

(73) Proprietor: **Politecnico di Milano**
**20133 Milano (IT)**

(72) Inventors:
- **METRANGOLO, Pierangelo**
  **I-20096 Pioltello (IT)**
- **RESNATI, Giuseppe**
  **I-20052 Monza (IT)**
- **CARCENAC, Yvan**
  **I-20133 Milano (IT)**

(74) Representative: **Perani, Aurelio et al**
**Perani & Partners**
**Piazza San Babila, 5**
**20122 Milano (IT)**

(56) References cited:
**US-A- 5 214 106 US-A- 6 002 055**

- **NAIR H. K. AND BURTON D. J: "Solvent effects on the reaction of perfluoroalkyl iodides and a,w-perfluoroalkyl diiodides with cadmium powder and dimethylcadmium" J. FLUOR. CHEM, vol. 60, 1993, pages 1-12, XP002504828**
- **ZAIMIS E. J.: "The synthesis of methonium compounds, their isolation from urine, and their photometric determination" BRIT. J. PHARMACOL., vol. 5, 1950, pages 424-430, XP002504829 cited in the application**
- **MCKENZIE D. C. ET AL.: "Dynamic and classical light scattering by bolaform micelles: comparison with normal micelles" J. PHYS. CHEM., vol. 91, no. 22, 1987, pages 5709-5713, XP002504830**
- **DANG TUAN A ET AL: "LASER MASS SPECTROMETRY OF DIQUATERNARY AMMONIUM SALTS." ANALYTICAL CHEMISTRY 1984 MAY, vol. 56, no. 6, May 1984 (1984-05), pages 866-871, XP002504831**
- **LEE C. AND JONES T.: "Molecular conformation-activity relationship of decamethonium congeners" BRIT. J. ANAESTHESIA, vol. 88, no. 5, 2002, pages 692-699, XP002504832**
- **FIORI A. AND MARIGO M.: "A method for the detection of d-tubocarine, gallamine, decamethonium and succinylcholine in biological materials" JOURNAL OF CHROMATOGRAPHY, vol. 31, 1967, pages 171-176, XP002504833**
- **FOX D B ET AL: "Perfluorocarbon-hydrocarbons self-assembly: halogen bonding mediated intermolecular recognition" JOURNAL OF FLUORINE CHEMISTRY, ELSEVIER, NL, vol. 125, no. 2, 2 February 2004 (2004-02-02), pages 271-281, XP004486530 ISSN: 0022-1139**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 181 082 B1

- GATTUSO ET AL: "Dipyridinocalixcrown/ diiodoperfluorocarbo n binary host systems for CsI: structural studies and fluorous phase extraction of caesium" TETRAHEDRON, ELSEVIER SCIENCE PUBLISHERS, AMSTERDAM, NL, vol. 63, no. 23, 4 May 2007 (2007-05-04), pages 4951-4958, XP022060064 ISSN: 0040-4020
- CASNATI A ET AL: "Molecular and supramolecular homochirality: Enantiopure perfluorocarbon rotamers and halogen-bonded fluorous double helices" ANGEWANDTE CHEMIE - INTERNATIONAL EDITION 20060313 WILEY-VCH VERLAG DE, vol. 45, no. 12, 13 March 2006 (2006-03-13), pages 1915-1918, XP002504834

## Description

The present invention concerns a process for the purification of α,ω-diiodoperfluorinated compounds and several pure α,ω-diiodoperfluorinated compounds thus obtained.

The α,ω-diiodoperfluorinated compounds constitute a class of compounds of interest for the fluorine chemical field, in particular as chain transfer reagents for the production of fluoroelastomers or polymerisation of fluorinated vinyl monomers.

US 5,214,106 relates to a cured fluoroelastomer composition prepared from, *inter alia,* an α,ω-perfluoroalkane selected from I-$(CF_2)_m$-I, wherein m = 3,4,6,8,12 and 12.

Fox BF et al, Journal of Fluorine Chemistry 125 (2004) 271 - 281, Gattuso G et al, Tetrahedron 63 (2007) 4951-4958 and Casnati A et al, Angew. Chem. Int. 2006, 45, 1915-1918 disclose the use of perfluorocarbon compounds in methods for sequestering and extracting $Cs^+$, $Ba^{2+}$ or $K^+$ from an aqueous phase.

From the US patents 6,150,565 and 6,002,055, processes for the preparation of dihaloperfluoroalkanes of formula X-$(CF_2)_n$-Y are known wherein n is an integer from 2 to 6 and X and Y are each independently I, Br or Cl. By following the teaching of these patents, yields of about 70% of products consisting of mixtures of different dihaloperfluoroalkanes can be obtained, in variable ratio from each other (for example, C2:C4:C6:C8:C10=7.29:48.27:29.68: 10.50:3.64 in mol%, where the abbreviations C2, C4, C6, C8, and C10 stand for tetrafluoro-1,2-diiodoethane, octafluoro-1,4-diiodobutane, dodecafluoro-1,6-diiodohexane, hexadeca-fluoro-1,8-diiodooctane, and icosafluoro-1,10-diiodo-decane, respectively), as pointed out by the results of the gas chromatography analysis carried out for each described embodiment.

From the US patent 4,731,170, the preparation of α,ω-dihaloperfluoroalkanes of formula X-$(CF_2CF_2)_n$-Y is known, wherein n is an integer from 2 to 6, through a telomerisation reaction. Particularly, in Example 1, it is specified that the compound I-$(CF_2CF_2)_2$-I is obtained by telomerisin.g $C_2F_4$ with I-$(C_2F_4)$-I and upon subsequent fractional distillation. In the same manner, I-$(CF_2CF_2)_3$-I in Example 3 was obtained.

Furthermore, from US patent 6,825,389, a process for preparing α,ω-diiodoperfluoroalkanes of formula I-$(CF_2CF_2)_n$-I is known wherein n is an integer from 2 to 6, obtained by reaction between 1,2-diiodoperfluoroethane and tetrafluoroethylene. In this way, mixtures of α,ω-diiodoperfluoroalkanes are obtained, from which small amounts of iodine must be separated.

In the case of short-chain α,ω-diiodoperfluoroalkanes, compounds of formula I-$(CF_2CF_2)_m$-I are commercially available wherein m is from 1 to 4 with high purity. Particularly, the following compounds are known:

- tetrafluoro-1,2-diiodoethane of formula I-$(CF_2CF_2)$-I (98% purity)
- octafluoro-1,4-diiodobutane of formula I-$(CF_2CF_2)_2$-I (98% purity)
- dodecafluoro-1,6-diiodohexane of formula I-$(CF_2CF\text{-}_2)_3$-I (96% purity)
- hexadecafluoro-1,8-diiodooctane of formula I-$(CF_2CF_2)_4$-I (98% purity).

Said compounds are obtained through known preparation processes and are purified through subsequent known purification methods, typically through fractional distillation. It is known that the use of fractional distillation in some cases allows to achieve very high purity grades, but anyhow in unsuitably poor yields. In addition, the same involves a complex and costly preparation, requires a lot of time and consumes much energy.

On the other hand, in the case of compounds of formula I-$(CF_2CF_2)_m$-I wherein m is higher than 4, as also disclosed in the prior art patents abovementioned, only mixtures thereof in variable ratios are known. In fact, the separation of such compounds by means of known separation methods, thus also by means of fractional distillation, has proven to be complex, difficult and scarcely effective.

Nair et al, Journal of Fluorine Chemistry, 60 (1993) 1-12 investigate the effects of solvents on the reaction of perfluoroalkyl iodides and α,ω-perfluoroalkyl didiodides with cadmium powder and dimethylcadmium and disclose the separation of diiodides with more than 12 $CF_2$ units by sublimation.

The object of the present invention is therefore to provide a process for the purification of α,ω-diiodoperfluorinated compounds having a number of carbon atoms of 2 to 20 which allows to obtain such compounds both in high yields and with optimal purity.

Such an object is achieved by a process of purification of α,ω-diiodoperfluorinated compounds as indicated in claim 1. Further advantages and preferred embodiments of the invention are indicated in the dependent claims.

Therefore, the invention relates to a process of purification of α,ω-diiodoperfluorinated compounds comprising contacting an impure α,ω-diiodoperfluorinated compound with a suitable sequestering agent, thus forming an adduct and separating the α,ω-diiodoperfluorinated compound from the adduct itself.

By the term "impure" in the present invention, it is intended a chemical compound having a purity of less than 95%, i.e. a compound which has impurities. By the term "impurities" in the present invention, it is intended both unreacted reagents and by-products of the process selected for the preparation of α,ω-diiodoperfluoroalkanes, such as for examples

iodine, solvent residues and decomposition products, and other α,ω-diiodoperfluoroalkanes different from that to be purified.

Further characteristics and advantages of the invention will be clear from the following detailed description made with reference to the exemplifying and non-limiting embodiments of the invention and to the attached figures, wherein:

- Figure 1 shows the crystalline structure of the adduct of the invention obtained according to Example 1;
- Figure 2 shows the crystalline structure of the adduct of the invention obtained according to Example 2;
- Figure 3 shows the crystalline structure of the adduct of the invention obtained according to Example 3;
- Figure 4 shows the crystalline structure of the adduct of the invention obtained according to Example 8.

Hence, the present invention concerns a process of purification of a α,ω-diiodoperfluorinated compound of formula $I\text{-}(CF_2CF_2)_m\text{-}I$ comprising the steps of:

a) providing an impure α,ω-diiodoperfluorinated compound,
b) contacting the impure α,ω-diiodoperfluorinated compound of step a) with a sequestering agent of formula $[CH_3)_3N\text{-}(CH_2)_n\text{-}N(CH_3)_3]^{2+}\cdot 2I^-$thus forming an adduct with the sequestering agent, and
c) separating the α,ω-diiodoperfluorinated compound from the adduct of step b), wherein

m is an integer from 1 to 10,
n=2m+6.

Step a) provides that an impure α,ω-diiodoperfluorinated compound of formula $I\text{-}(CF_2CF_2)_m\text{-}I$ is provided.

Such an impure compound can be a compound having impurities of about 2-5%, as previously indicated for the case of a number of carbon atoms less than 8, or such an impure compound can be a compound having, as impurities, mixtures of other α,ω-diiodoperfluorinated compounds not of interest with even high percentages, i.e. of about 60%, obtained according to methods of the prior art.

Step b) provides that the α,ω-diiodoperfluorinated compound of formula $I\text{-}(CF_2CF_2)_m\text{-}I$ of step a) contacts with a sequestering agent of formula

$$[(CH_3)_3N\text{-}(CH_2)_n\text{-}N(CH_3)_3]^{2+}\cdot 2I^-$$

wherein m is an integer from 1 to 10 and n=2m+6.

Preferably, in step b) of the process according to the invention, the sequestering agent is in an amount equimolar to the α,ω-diiodoperfluorinated compound to be purified.

The sequestering agent of the present invention can be synthesized according to methods known in literature.

For example, from the publication of J. Zhang et al. (J Mol. Struct., 2003, 660, 119-129) decamethonium congeners are known; particularly, compounds are known of formula:

$$(CH_3)_3N^+\text{-}(CH_2)_m\text{-}N^+(CH_3)_3;$$

wherein m ranges from 5 to 18.

Dang T.A. et al, Analytical chemistry, vol. 56, no. 6, May 1984. pp 866-871 report and discuss the results of laser mass spectrometry of diquatemary ammonium salts of formula [(R1)(R2)(R3)-A-B-A-(R4)(R5)(R6)] wherein B is, inter alia $(CH_2)_8$ and R1-R6 are Me. Dimethonium compounds are also disclosed by Lee C et al in Brit. J. Anaesthesia, sol.88, no. 5, 2001, pp 692-699.

For the synthesis of such sequestering agents, the methods described in the publication titled "The synthesis of methonium compounds, their isolation from urine, and their photometric determination", by Zaimis, Eleanor J. Natl. Inst. Med. Research, London, British Journal of Pharmacology and Chemotherapy (1950), 5 - 424-30, can be suitable.

For the purification of decamethonium, reference can also be made to Fiori A. et al, Journal of Chomatography, vol. 31, 1967, pp 171-176.

The sequestering agent according to the present invention can be provided in solid powder form, in a suitable solvent solution or bound or sorbed on a suitable support. Such a suitable support can be an organic polymer support, which can optionally be coated with a coating having sorbing capacities, or can be an inorganic particulate, which can optionally be coated with a coating having sorbing capacities. Said an inorganic particulate can be, for example, a molecular sieve, or an inorganic particulate trapped in porous polymer fabric. If the sequestering agent is in solid powder form or in bonded form or sorbed on a suitable support, said agent can be provided on a bed, column or tube.

In step b) of the present invention, the α,ω-diiodoperfluorinated compound of formula $I\text{-}(CF_2CF_2)_m\text{-}I$ forms an adduct with the sequestering agent as described above wherein n=2m+6.

Without wishing to be bound by any theory, it is believed that the sequestering agent is able to form a dimensionally suitable cavity for hosting the α,ω-diiodoperfluorinated compound having a chain of m atoms, owing to interactions between the anions $I^-$ of the sequestering agent and the atoms 1 of the α,ω-diiodoperfluorinated compound. Said cavity allows the α,ω-diiodoperfluorinated compound to be purified to be high selectively sequestered by the sequestering agent, even in the presence of other α,ω-diiodoperfluorinated compounds both with longer chain and with shorter chain.

As will be clear from the following examples, the formed adduct is separated from the reaction mixture by precipitation as a crystalline solid composed of the sequestering agent and the sole α,ω-diiodoperfluorinated compound to be purified.

Step c) provides the separation of the α,ω-diiodoperfluorinated compound from the adduct formed in the previous step b). Once the adduct is filtered and washed with a suitable solvent, for example $CH_2Cl_2$, the separation of the α,ω-diiodoperfluorinated compound from the adduct itself is performed.

Such a separation is carried out by vacuum sublimation, optionally at temperatures greater than room temperature, with recovery of the α,ω-diiodoperfluorinated compound via recondensation at low temperature. In fact, the α,ω-diiodoperfluorinated compounds are volatile, whereas the sequestering agent, as an organic salt, has an extremely high sublimation point. On the basis of this different characteristic, the adduct crystals are then placed in a chamber wherein the vacuum is applied. Long-chain α,ω-diiodoperfluorinated compounds, which are less volatile, require the application of a greater vacuum for sublimating than the short-chain α,ω-diiodoperfluorinated compounds. Preferably, such a vacuum is in the range of 10 mm Hg - 0.1 mm Hg. More preferably, during the application of the vacuum, the temperature of the chamber is room temperature. Advantageously, in the case of long-chain α,ω-diiodoperfluorinated compounds, the temperature of said chamber can be increased to about 60°C, in order to promote the quantitative sublimation of such compounds. Advantageously, for these purposes, the adduct crystals can be previously milled in order to increase the exposed surface area as much as possible.

The sublimation chamber is advantageously connected to at least one cooling trap for the recondensation of the sublimated α,ω-diiodoperfluorinated compound. Preferably, such a trap has a temperature of at least -60°C. In the case of short-chain α,ω-diiodoperfluorinated compounds, and thus more volatile compounds, the temperature can be further lowered, advantageously to the temperature of liquid nitrogen, i.e. to about -200°C. Even more advantageously, the sublimation chamber is connected to a series of cooling traps each having a temperature lower than the previous one, in order to increase the recovery yield of the α,ω-diiodoperfluorinated compound via recondensation at decreasing temperatures.

Alternatively, the separation of the α,ω-diiodoperfluorinated compound from the adduct can be carried out via solid/liquid chromatographic separation.

As will also be evident from the following examples, without wishing to be bound by any theory, it is believed that the $I\cdots I^-$ interactions present in the adduct between the α,ω-diiodoperfluorinated compound and the sequestering agent are such as to make advantageously reversible the formation of the adduct itself. Therefore, once formed, the adduct can be treated as described above for step c), so as to quantitatively release the sequestered α,ω-diiodoperfluorinated compound. Moreover, the fact that, in step b), the sequestering agent quantitatively and selectively sequesters the compound of interest and then, in step c), likewise quantitatively releases the same, allows to obtain advantageously high yields of pure α,ω-diiodoperfluorinated compound. In any case, the yield of step c), i.e. the yield of the separation of the α,ω-diiodoperfluorinated compound from the adduct results close to 100%.

The process of the present invention therefore allows to selectively and quantitatively sequester the α,ω-diiodoperfluorinated compound of interest and purify the same by separating it from the sequestering agent, thus obtaining, in significantly high yields, an α,ω-diiodoperfluorinated compound having purity of at least 95%. Preferably, such a process allows to obtain, in significantly high yields, an α,ω-diiodoperfluorinated compound having purity of at least 99%. Even more preferably, such a process allows to obtain, in significantly high yields, an α,ω-diiodoperfluorinated compound having purity of 100%.

Furthermore, such a process is extremely advantageous from the point of view of the implementation and production advantage, through simple and economical steps, since the adduct precipitates and separates at room temperature and the sequestering agent can be reused.

In another aspect, the invention concerns an adduct of a α,ω-diiodoperfluorinated compound of formula $I\text{-}(CF_2CF_2)_m\text{-}I$ and a sequestering agent of formula

$$[(CH_3)_3N\text{-}(CH_2)_n\text{-}N(CH_3)_3]^{2+}\cdot 2I^-$$

wherein

m is an integer from 1 to 10, and n=2m+6, as claimed in claim 4.

It has to be understood that all aspects related to the sequestering agent described above for the purification process of a α,ω-diiodoperfluorinated compound of the invention, even advantageous and preferred in terms of product characteristics, are the same for the adduct.

Such an adduct is in solid form and is composed of the sequestering agent and the sole α,ω-diiodoperfluorinated compound to be purified. In this way, a highly selective and quantitative sequestration of the α,ω-dilodcrperfluorinated compound from the impure starting compound is achieved. In the adduct, the α,ω-diiodoperfluorinated compound is very stable, whereas it is known that perfluorinated compounds are very volatile and tend to rapidly degrade. Therefore, the formation of the adduct is extremely advantageous and convenient not only for the purposes of purification of the α,ω-diiodoperfluorinated compounds, but also for example for trapping such compounds in stable matrices for their improved preservation. Another example can be the use in processes wherein the α,ω-diiodoperfluorinated compound must be supplied in a constant amount over time. In this case, the adduct crystals can be placed directly in the reaction chamber and progressively heated so that the α,ω-diiodoperfluorinated compound is released at the required flow rate.

Some examples now follow, here provided as exemplifying and non-limiting, of the purification process of α, ω-diiodoperfluoroalkanes according to the invention.

**Example 1**

**Purification of tetrafluoro-1,2-diiodoethane (m=1) (via solution)**

83 mg of a commercial mixture of α,ω-diiodoperfluorinated compounds (sold by Apollo Scientific Ltd.) composed of 25% tetrafluoro-1,2-diiodoethane (m=1), 25% octafluoro-1,4-tetraiodobutane (m=2), 25% dodecafluoro-1,6-diiodohexane (m=3) and 25% hexadecafluoro-1,8-diiodooctane (m=4) were solubilized in 1 ml of $CHCl_3$, and separately 20 mg of sequestering agent of formula $(CH_3)_3N^+-(CH_2)_8-N^+(CH_3)_3 \cdot 2I^-$, i.e. octamethonium iodide, were solubilized in 1 ml of $CH_3OH$.

The two solutions were then mixed in a test tube that was then closed. After about 2 hours, the formation of a solid white precipitate was observed, which was filtered, washed twice with $CCl_4$ and dried under vacuum. The results of the performed analyses, as reported below, confirmed that this was an adduct of octamethonium iodide and tetrafluoro-1,2-diiodoethane.

The tetrafluoro-1,2-diiodoethane was then separated from the sequestering agent by sublimation of the crystalline adduct under vacuum at a temperature of about 30˚C and recovered via recondensation at a temperature of about -198˚C.

100% pure tetrafluoro-1,2-diiodoethane was then obtained.

The following tests were carried out on the crystalline adduct:

- melting point: 201˚C;
- IR ($cm^{-1}$, selective bands):
    - pure octamethonium iodide: 3012, 2929, 2857, 1475, 1465, 966, 955, 921, 907;
    - adduct of octamethonium iodide and tetrafluoro-1,2-diiodoethane: 3011, 2945, 2872, 2854, 1477, 1405, 1128, 1090, 950, 905, 693.
- $^{19}F$ NMR (470.6 MHz, $CD_3OD$, 0.002 M):
    - adduct of octamethonium iodide and tetrafluoro-1,2-diiodoethane: $\Delta\delta_F$= 0.02.

On the adduct crystal, an XRD analysis was carried out which confirmed the exclusive presence of octamethonium iodide and tetrafluoro-1,2-diiodoethane. In fact, Figure 1 represents the crystalline structure of the adduct wherein the molecules of octamethonium iodide (the carbon and nitrogen atoms are light grey coloured and the hydrogen atoms white) alternate with the molecules of tetrafluoro-1,2-diiodoethane (the carbon atoms are light grey coloured, the hydrogen atoms white and the iodine atoms dark grey) and the iodide atoms (dark grey coloured). Furthermore, it was observed that, in crystalline state, octamethonium iodide and tetrafluoro-1,2-diiodoethane form alternating parallel layers since the tetrafluoro-1,2-diiodoethane occupies the cavity defined by the spacer chain between the two nitrogen atoms of the octamethonium iodide and the two iodides of the same, due to the iodide-iodine interactions generated and which in Figure 1 are depicted with a dashed line.

Thus, the following crystallographic measurements are reported:

| Adduct | Distance A | Distance B | Δ (B-A) |
|---|---|---|---|
| | $N(CH_2)_nN$ 11.343 Å | $I^- \cdots I(CF_2CF_2)_mI \cdots I^-$ 12.142 Å | 0.799 Å |

As can be observed, in the adduct crystal, the difference Δ (B - A) between the distance B between the iodine atoms aligned with the tetrafluoro-1,2-diiodoethane molecule, as shown in Figure 1, and the distance A between the nitrogen atoms belonging to a same octamethonium molecule is equal to 0.799 Å and significant of the existence of said interactions $I \cdots I^-$.

Specifically, therefore, the relation n=2m+6 allows to select the suitable sequestering agent, i.e. the sequestering agent having a distance A between the nitrogen or phosphorus atoms appropriately proportioned to the size of the α, ω-diiodoperfluorinated compound to be purified, which is thus advantageously hosted in the cavity generated by the chain B of the sequestering agent and the iodine atoms of the same, as is clearly shown in Figure 1.

In fact, without wishing to be bound by any theory, it is believed that the high melting point and the high stability of the adduct at the solid state are due to the crystalline packing observed, which can explain the selectivity of the sequestering agent, suitably selected according to the teachings of the present invention, based on the α,ω-diiodoperfluorinated compound to be purified, even in the presence of other α,ω-diiodoperfluorinated compounds with value m much closer to that of the compound to be purified. Such a selectivity is expressed in qualitative terms, i.e. according to the relation n=2m+6, and in quantitative terms, since under conditions of molar excess of the sequestering agent with respect to the α,ω-diiodoperfluorinated compound, the quantitative precipitation of the crystalline adduct corresponding to the relation n=2m+6 is first observed, and then, once the α,ω-diiodoperfluorinated compound to be purified is exhausted, the adduct of the sequestering agent having a different m much more slowly starts to form. Particularly, it was observed that an adduct will start to form with that α,ω-diiodoperfluorinated compound having a different m, which, with respect to others equally present in solution, generate crystals having highest melting points.

On the pure tetrafluoro-1,2-diiodoethane thus obtained, the following tests were carried out:

- IR ($cm^{-1}$, selective bands):
  - pure tetrafluoro-1,2-diiodoethane: 1147, 1096, 973, 834, 89;
- $^{19}F$ NMR (470.6 MHz, $CD_3OD$, 0.002 M):
  - pure tetrafluoro-1,2-diiodoethane: $\delta_F$ = -56.24.

Also the gas chromatography tests confirmed that this was only 100% tetrafluoro-1,2-diiodoethane.

Without wishing to be bound by any theory, it is believed that the interactions $I \cdots I^-$observed in the adduct are such to make advantageously reversible the formation of the adduct itself. Therefore, once formed, the adduct can be treated as described for step c), so as to quantitatively release the sequestered α,ω-diiodoperfluorinated compound. Furthermore, the fact that the sequestering agent quantitatively and selectively sequesters the compound of interest and then quantitatively releases it, allows to obtain advantageously high yields of pure α,ω-diiodoperfluorinated compound.

The process according to the present invention therefore allows to obtain extremely high yields of pure tetrafluoro-1,2-diiodoethane from a mixture of different α,ω-diiodoperfluorinated compounds. Therefore, even more so, the process of the invention is effective in the purification of the same compound already available on the market with high purities, as also shown in Example 9.

**Example 2**

**Purification of octafluoro-1,4-diiodobutane (m=2) (via solution)**

79 mg of a commercial mixture of α,ω-diiodoperfluorinated compounds (sold by Apollo Scientific Ltd.) composed of 25% tetrafluoro-1,2-diiodoethane (m=1), 25% octafluoro-1,4-tetraiodobutane (m=2), 25% dodecafluoro-1,6-diiodohexane (m=3) and 25% hexadecafluoro-1,8-diiodooctane (m=4) were solubilized in 1 ml of $CHCl_3$, and separately 20 mg of sequestering agent of formula $(CH_3)_3N^+\text{-}(CH_2)_{10}\text{-}N^+(CH_3)_3 \cdot 2I^-$, i.e. decamethonium iodide, were solubilized in 1 ml of $CH_3OH$.

The two solutions were then mixed in a test tube which was then closed. After about 2 hours, the formation of a solid white precipitate was observed, which was filtered, washed twice with $CCl_4$ and dried under vacuum. The results of the performed tests, as reported below, confirmed that this was an adduct of decamethonium iodide and octafluoro-1,4-diiodobutane.

The octafluoro-1,4-diiodobutane was then separated by the sequestering agent by sublimation from the crystalline adduct under vacuum at temperature of about 40˚C and recovered by recondensation at a temperature of about -198˚C.

100% pure octafluoro-1,4-diiodobutane was then obtained.

The following tests were performed on the crystalline adduct:

- melting point: 230˚C;
- IR ($cm^{-1}$, selective bands):
  - pure decamethonium iodide: 3002, 2923, 2859, 1628, 1492, 1479, 964, 950, 910, 714;
  - adduct of decamethonium iodide and octafluoro-1,4-diiodobutane: 3010, 2941, 2870, 1475, 1405, 1184, 1123, 1040, 961, 761.
- $^{19}F$ NMR (470.6 MHz, $CD_3OD$, 0.002 M):
  - adduct of decamethonium iodide and octafluoro-1,4-diiodobutane: $\Delta\delta_{(ICF2CF2)2} = 0.02$, $\Delta\delta_{(ICF2C\mathit{F}2)_2} = 0.00$.

An XRD test was performed on the adduct crystal which confirmed the exclusive presence of decamethonium iodide and octafluoro-1,4-diiodobutane. In fact, Figure 2 represents the crystalline structure of the adduct wherein the molecules of decamethonium iodide (the carbon and nitrogen atoms are light grey coloured) alternate with the molecules of octafluoro-1,4-diiodobutane (the carbon atoms are light grey coloured, the hydrogen atoms white and the iodine atoms dark grey) and with the iodide atoms (dark grey coloured). Moreover, it was observed that, at the crystalline state, decamethonium iodide and octafluoro-1,4-diiodobutane form alternating parallel layers, since octafluoro-1,4-diiodobutane occupies the cavity defined by the spacer chain between the two nitrogen atoms of the decamethonium iodide and the two iodides of the same, due to the iodide-iodine interactions generated.

Thus, the following crystallographic measurements are reported:

| Adduct | Distance A | Distance B | Δ (B-A) |
|---|---|---|---|
| | $\underbrace{N(CH_2)_nN}_{13.617\ Å}$ | $\underbrace{I^- \cdots I(CF_2CF_2)_mI \cdots I^-}_{14.483\ Å}$ | 0.866 Å |

As can be observed, the adduct crystal, the difference Δ (B - A) between the distance B between the iodide atoms aligned with the molecule of octafluoro-1,4-diiodobutane, as shown in Figure 2, and the distance A between the nitrogen atoms belonging to the same decamethonium molecule is equal to 0.866 Å and significant of the existence of said interactions $I \cdots I^-$.

The following tests were performed on the pure octafluoro-1,4-diiodobutane thus obtained:

- IR ($cm^{-1}$, selective bands):
  - pure octafluoro-1,4-diiodobutane: 1190, 1130, 1039, 887, 763;
- $^{19}F$ NMR (470.6 MHz, $CD_3OD$, 0.002 M):
  - pure octafluoro-1,4-diiodobutane: $\delta$ = -63.80 $(IC\mathit{F}_2CF_2)_2$, -112.02 $(ICF_2C\mathit{F}_2)_2$.

The gas chromatography tests also confirmed that this was only 100% octafluoro-1,4-diiodobutane.

**Example 3**

**Purification of dodecafluoro-1,6-diiodohexane (m=3) (via solution)**

75 mg of a commercial mixture of $\alpha,\omega$-diiodoperfluorinated compounds (sold by Apollo Scientific Ltd.) composed of 25% tetrafluoro-1,2-diiodoethane (m=1), 25% octafluoro-1,4-tetraiodobutane (m=2), 25% dodecafluoro-1,6-diiodohexane (m=3) and 25% hexadecafluoro-1,8-diiodooctane (m=4) were solubilized in 1 ml of $CHCl_3$, and separately 20 mg of sequestering agent of formula $(CH_3)_3N^+\text{-}(CH_2)_{12}\text{-}N^+(CH_3)_3\cdot 2I^-$, i.e. dodecamethonium iodide, were solubilized in 1 ml of $CH_3OH$.

The two solutions were then mixed in a test tube which was then closed. After about 2 hours, the formation of a solid white precipitate was observed, which was filtered, washed twice with $CCl_4$ and dried under vacuum. The results of the performed tests, as reported below, confirmed that this was an adduct of dodecamethonium iodide and dodecafluoro-1,6-diiodohexane.

The dodecafluoro-1,6-diiodohexane was then separated from the sequestering agent by sublimation of the crystalline adduct under vacuum at a temperature of about 50°C and recovered by recondensation at a temperature of about -198°C.

100% pure dodecafluoro-1,6-diiodohexane was then obtained.

The following tests were conducted on the crystalline adduct:

- melting point: 226°C;
- IR ($cm^{-1}$, selective bands):
    - pure dodecamethonium iodide: 3002, 2914, 2851, 1483, 1464, 973, 939, 916, 731;
    - adduct of dodecamethonium iodide and dodecafluoro-1,6-diiodohexane: 3010, 2941, 2867, 1475, 1203, 1 i41, 1125, 1081, 963, 909, 731.
- $^{19}F$ NMR (470.6 MHz, $CD_3OD$, 0.002 M):
    - adduct of dodecamethonium iodide and dodecafluoro-1,6-diiodohexane:

$$\Delta\delta_{(IC\mathit{F2}CF2CF2)2} = 0.08,\ \Delta\delta_{(ICF2C\mathit{F2}CF2)2} = 0.01,\ \Delta\delta_{(ICF2CF2C\mathit{F2})2} = 0.00.$$

An XRD test was performed on the adduct crystal which confirmed the exclusive presence of decamethonium iodide and dodecafluoro-1,6-diiodohexane. In fact, Figure 3 represents the crystalline structure of the adduct in which the molecules of dodecamethonium iodide (the carbon and nitrogen atoms are light grey coloured and the hydrogen atoms white) alternate with the molecules of dodecafluoro-1,6-diiodohexane (carbon atoms are light grey coloured, hydrogen atoms white and the iodine atoms dark grey) and with the iodide atoms (dark grey coloured). Moreover, it was observed that, at the crystalline state, decamethonium iodide and dodecafluoro-1,6-diiodohexane form alternating parallel layers, since the dodecafluoro-1,6-diiodohexane occupies the cavity defined by the spacer chain between the two nitrogen atoms of the dodecamethonium iodide and the two iodides of the same, due to the iodide-iodine interactions generated.

Thus, the following crystallographic measurements are reported:

| Adduct | Distance | Distance B | Δ(B-A) |
|---|---|---|---|
| | $\underbrace{N(CH_2)_nN}_{16.395\ Å}$ | $\underbrace{I^-\cdots I(CF_2CF_2)_mI\cdots I^-}_{17.083\ Å}$ | 0.688 Å |

As can be observed, in the adduct crystal, the difference Δ (B - A) between the distance B between the iodide atoms aligned with the molecule of dodecafluoro-1,6-diiodohexane, as shown in figure 1, and the distance A between the nitrogen atoms belonging to the same dodecamethonium molecule is equal to 0.688 Å and significant of the existence of said interactions $I\cdots I^-$.

The following tests were performed on the pure dodecafluoro-1,6-diiodohexane:

- IR ($cm^{-1}$, selective bands):

  - pure dodecafluoro-1,6-diiodohexane: 1190, 1130, 1039, 887, 763;

- $^{19}F$ NMR (470.6 MHz, $CD_3OD$, 0.002 M):

  - pure dodecafluoro-1,6-diiodohexane: $\delta$ = -63.80 $(ICF_2CF_2)_2$, -112.02 $(ICF_2CF_2)_2$.

The gas chromatography tests also confirmed that this was only 100% dodecafluoro-1,6-diiodohexane.

**Example 4**

**Purification of hexadecafluoro-1,8-diiodooctane (m=4) (via solution)**

35 mg of a commercial mixture of $\alpha,\omega$-diiodoperfluorinated compounds (sold by Apollo Scientific Ltd.) composed of 25% tetrafluoro-1,2-diiodoethane (m=1), 25% octafluoro-1,4-tetraiodobutane (m=2), 25% dodecafluoro-1,6-diiodohexane (m=3) and 25% hexadecafluoro-1,8-diiodooctane (m=4) were solubilized in 1 ml of $CHCl_3$, and separately 10 mg of sequestering agent of formula $(CH_3)_3N^+-(CH_2)_{14}-N^+(CH_3)_3\cdot 2I^-$, i.e. tetradecamethonium iodide, were solubilized in 1 ml of $CH_3OH$.
The two solutions were then mixed in a test tube that was then closed. After about 2 hours, the formation of a solid white precipitate was observed, which was filtered, washed twice with $CCl_4$ and dried under vacuum. The results of the performed tests confirmed that this was an adduct of tetradecamethonium iodide and hexadecafluoro-1,8-diiodooctane.
The hexadecafluoro-1,8-diiodooctane was then separated by the sequestering agent by sublimation from the crystalline adduct under vacuum at temperature of about 60˚C and recovered by recondensation at a temperature of about -198˚C.
100% pure hexadecafluoro-1,8-diiodooctane was then obtained.
The following tests were performed on the crystalline adduct:

- melting point: 230˚C;
- IR ($cm^{-1}$, selective bands):

  - pure tetradecamethonium iodide: 3004, 2917, 2852, 1482, 1464, 972, 951, 921, 897, 733;
  - adduct of tetradecamethonium iodide and hexadecafluoro-1,8-diiodooctane: 3010, 2940, 2865, 1475, 1210, 1146, 1105, 1056, 959, 905, 827, 731.

- $^{19}F$ NMR (470.6 MHz, $CD_3OD$, 0.002 M):

  - adduct of hexadecafluoro-1,8-diiodooctane and hexadecafluoro-1,8-diiodooctane:

$$\Delta\delta_{(IC\mathit{F}2CF2CF2CF2)2} = 0.10,\ \Delta\delta_{(ICF2C\mathit{F}2CF2CF2)2} = 0.01,\ \Delta\delta_{(ICF2CF2C\mathit{F}2CF2)2} = 0.00,$$
$$\Delta\delta_{(ICF2CF2CF2C\mathit{F}2)2} = 0.00.$$

An XRD test was performed on the adduct crystal which confirmed the exclusive presence of tetradecamethonium iodide and hexadecafluoro-1,8-diiodooctane. Furthermore, it was observed that, at the crystalline state, tetradecamethonium iodide and hexadecafluoro-1,8-diiodooctane form alternating parallel layers and that the hexadecafluoro-1,8-diiodooctane occupies the cavity defined by the spacer chain between the two nitrogen atoms of the decamethonium iodide and the two iodides of the same, due to the iodide-iodine interactions generated.
The following tests were performed on the pure hexadecafluoro-1,8-diiodooctane:

- IR ($cm^{-1}$, selective bands):

  - pure hexadecafluoro-1,8-diiodooctane: 1203, 1145, 1112, 1090, 1055, 833;

- $^{19}F$ NMR (470.6 MHz, $CD_3OD$, 0.002 M):

  - pure hexadecafluoro-1,8-diiodooctane: $\delta$ = -64.71 $(IC\mathit{F}_2CF_2CF_2CF_2)_2$, -113.01 $(ICF_2C\mathit{F}_2CF_2CF_2)_2$, -120.,20

$(ICF_2CF_2CF_2CF_2)_2$, -121.05 $(ICF_2CF_2CF_2CF_2)_2$.

The gas chromatography tests also confirmed that this was only 100% hexadecafluoro-1,8-diiodooctane.

**Example 5**

**Purification of hexadecafluoro-1,8-diiodooctane (m=4) (via solution)**

47 mg of a commercial mixture of $\alpha,\omega$-diiodoperfluorinated compounds (obtained by means of fractional distillation and provided by Solvay Solexis S.p.A.) composed of 29.3% hexadecafluoro-1,8-diiodooctane (m=4), 41.9% icosafluoro-1,10-diiodododecane (m=5), 20.5% tetracosafluoro-1,12-diiodododecane (m=6), 6.6% octacosafluoro-1,14-diiodotetradecane (m=7) and 1.6% dotriacontafluoro-1,16-diiodohexadecane (m=8) were solubilized in 3 ml of $CFCl_2CF_2Cl$, and separately 9 mg of sequestering agent of formula $(CH_3)_3N^+-(CH_2)_{14}-N^+(CH_3)_3 \cdot 2I^-$, i.e. tetradecamethonium iodide, were solubilized in 1 ml of $CFCl_2CF_2Cl$.

The two solutions were then mixed in a test tube which was then closed. After about 2 hours, the formation of a solid white precipitate was observed, which was filtered, washed twice with $CCl_4$ and dried under vacuum. The results of the performed tests, as reported below, confirmed that this was an adduct of tetradecamethonium iodide and hexadecafluoro-1,8-diiodooctane.

The hexadecafluoro-1,8-diiodooctane was then separated by the sequestering agent by sublimation from the crystalline adduct under vacuum at temperature of about 60°C and recovered by recondensation at a temperature of about -198°C.

100% pure hexadecafluoro-1,8-diiodooctane was then obtained.

The following tests were performed on the crystalline adduct:

- melting point: 230°C;
- IR ($cm^{-1}$, selective bands):
  - pure tetradecamethonium iodide: 3004, 2917, 2852, 1482, 1464, 972, 951, 921, 897, 733;
  - adduct of tetradecamethonium iodide and hexadecafluoro-1,8-diiodooctane: 3010, 2940, 2865, 1475, 1210, 1146, 1105, 1056, 959, 905, 827, 731.
- $^{19}F$ NMR (470.6 MHz, $CD_3OD$, 0.002 M):
  - adduct of tetradecamethonium iodide and hexadecafluoro-1,8-diiodooctane:

$$\Delta\delta_{(ICF2CF2CF2CF2)2} = 0.10,\ \Delta\delta_{(ICF2CF2CF2CF2)2} = 0.01,\ \Delta\delta_{(ICF2CF2CF2CF2)2} = 0.00,$$

$$\Delta\delta_{(ICF2CF2CF2CF2)2} = 0.00.$$

An XRD test was performed on the adduct crystal which confirmed the exclusive presence of tetradecamethonium iodide and hexadecafluoro-1,8-diiodooctane. Furthermore, it was observed that, at the crystalline state, tetradecamethonium iodide and hexadecafluoro-1,8-diiodooctane form alternating parallel layers, and that the hexadecafluoro-1,8-diiodooctane occupies the cavity defined by the spacer cavity between the nitrogen atoms of the decamethonium iodine and the two iodides of the same, due to the iodide-iodine interactions generated.

The following tests were performed on the pure hexadecalluoro-1,8-diiodooctane:

- IR ($cm^{-1}$, selective bands):
  - pure hexadecafluoro-1,8-diiodooctane: 1203, 1145, 1112, 1090, 1055, 833;
- $^{19}F$ NMR (470.6 MHz, $CD_3OD$, 0.002 M):
  - pure hexadecafluoro-1,8-diiodooctane: $\delta$ = -64.71 $(ICF_2CF_2CF_2CF_2)_2$, -113.01 $(ICF_2CF_2CF_2CF_2)_2$, -120.20 $(ICF_2CF_2CF_2CF_2)_2$, -121.05 $(ICF_2CF_2CF_2CF_2)_2$.

The gas chromatography tests also confirmed that this was only 100% hexadecafluoro-1,8-diiodooctane.

**Example 6**

**Purification of icosafluoro-1,10-diiododecane (m=5) (via solution)**

25 mg of a commercial mixture of α,ω-diiodoperfluorinated compounds (obtained by means of fractional distillation and provided by Solvay Solexis S.p.A.) composed of 29.3% hexadecafluom-1,8-diiodooctane (m=4), 41.9% icosafluoro-1,10-diiodododecane (m=5), 20.5% tetracosafluoro-1,12-diiodododecane (m=6), 6.6% octacosafluoro-1,14-diiodotetradecane (m=7) and 1.6% dotriacontafluoro-1,16-diiodohexadecane (m=8) were solubilized in 1 ml of $CCl_4$, and separately 6 mg of sequestering agent of formula $(CH_3)_3N^+$-$(CH_2)_{16}$-$N^+(CH_3)_3 \cdot 2I^-$, i.e. hexadecamethonium iodide, were solubilized in 1 ml of $CH_3OH$.

The two solutions were then mixed in a test tube which was then closed. After about 2 hours, the formation of a solid white precipitate was observed, which was filtered, washed twice with $CCl_4$ and dried under vacuum. The results of the performed tests confirmed that this was an adduct of hexadecamethonium iodide and icosafluoro-1,10-diiododecane.

The crystalline adduct has a melting point of 222˚C.

The icosafluoro-1,10-diiododecane was then separated from the sequestering agent by sublimation of the crystalline adduct under vacuum at a temperature of about 60˚C and recovered by recondensation at a temperature of about -198˚C.

The gas chromatography tests also confirmed that this was only 100% pure icosafluoro-1,10-diiododecane.

**Example 7**

**Purification of tetracosafluoro-1,12-diiodododecane (m=6) (via solution)**

50 mg of a commercial mixture of α,ω-diiodoperfluorinated compounds (obtained by means of fractional distillation and provided by Solvay Solexis S.p.A.) composed of 29.3% hexadecafluoro-1,8-diiodooctane (m=4), 41.9% icosafluoro-1,10-diiodododecane (m=5), 20.5% tetracosafluoro-1,12-diiodododecane (m=6), 6.6% octacosafluoro-1,14-diiodotetradecane (m=7) and 1.6% dotriacontafluoro-1,16-diiodohexadecane (m=8) were solubilized in 3 ml of $CFCl_2CF_2Cl$, and separately 5 mg of sequestering agent of formula $(CH_3)_3N^+$-$(CH_2)_{18}$-$N^+(CH_3)_3 \cdot 2I^-$, i.e. octadecamethonium iodide, were solubilized in 1 ml of $CFCl_2CF_2Cl$.

The two solutions were then mixed in a test tube, which was then closed. After about 2 hours, the formation of a solid white precipitate was observed, which was filtered, washed twice with $CCl_4$ and dried under vacuum. The results of the performed tests confirmed that this was an adduct of octadecamethonium iodide and tetracosafluoro-1,12-diiodododecane.

The tetracosafluoro-1,12-diiodododecane was then separated by the sequestering agent by sublimation from the crystalline adduct under vacuum at temperature of about 60˚C and recovered by recondensation at a temperature of about -198˚C.

The gas chromatography tests also confirmed that this was only 100% pure tetracosafluoro-1,12-diiodododecane.

**Example 8**

**Evaluation of the selectivity of the sequestering agent**

1. Preparation of an adduct of octamethonium iodide (n=8) and octafluoro-1,4-diiodobutane (m=2)

20 mg (1 eq) of octamethonium iodide were dissolved in about 0.5 ml of $CH_3OH$. Such a solution was poured into a test tube containing a solution of 18.8 mg (1 eq) of octafluoro-1,4-diiodobutane, obtained according to Example 2, in about 0.5 ml of $CHCl_3$. The open test tube was then placed in a cylindrical body containing paraffin oil. The bottle was then closed. After several days, the slow diffusion of the solvent in the bottle led to the precipitation of an adduct of octamethonium iodide and octafluoro-1,4-diiodobutane in the form of a white solid.

The following tests were performed on such a precipitate:

- melting point: 188˚C;
- IR ($cm^{-1}$, selective bands): 3015, 2929, 2862, 1488, 1450, 1178, 1128, 1042, 954, 910, 759.

The tests confirmed that this was a pure adduct of octamethonium iodide and octafluoro-1,4-diiodobutane. An XRD test was conducted on the adduct crystal which confirmed the exclusive presence of octamethonium iodide and

octafluoro-1,4-diiodobutane. In fact, Figure 4 shows the crystalline structure of the adduct in which the octamethonium iodide molecules (the carbon and nitrogen atoms are light grey colour and the hydrogen atoms are white) alternate with the octafluoro-1,4-diiodobutane molecules (the carbon atoms are light grey coloured, hydrogen atoms white and the iodine atoms dark grey).

Thus, the following crystallographic measurements are reported:

| Adduct | Distance A | Distance B | Δ (B - A) |
|---|---|---|---|
| | $N(CH_2)_nN$ 10.535 Å | $I^- \cdots I(CF_2CF_2)_mI \cdots I^-$ 14.105 Å | 3.570 Å |

As can be observed, in the adduct crystal, the difference Δ (B - A) between the distance B between the iodide atoms aligned with the octafluoro-1,4-diiodobutane molecule, as shown in Figure 4, and the distance A between the nitrogen atoms belonging to the same molecule of octamethonium is equal to 3.570 Å, thus much higher than that observed in Example 1 wherein the adduct was composed of the same sequestering agent with tetrafluoro-1,2-diiodoethane. Moreover, it can be observed that also the distance A between the nitrogen atoms in the case of Example 1 was much higher than the same distance in the case of the present adduct, for which the relation n=2m+6 is not observed. Without wishing to be bound by any theory, it is believed that the adduct of octamethonium iodide and octafluoro-1,4-diiodobutane has a different crystalline packing with respect to the crystalline packing observed in the case of Example 1. In fact, in this case, the distance A between the nitrogen atoms belonging to the same octamethonium molecule is insufficient for generating a cavity suitable for hosting ottafluoro-1,4-diiodobutane. Therefore, even if formed, the adduct has a different crystalline structure.

<u>2. Test of selectivity of the octamethonium iodide</u>

20 g of octamethonium iodide and octafluoro-1,4-diiodobutane adduct thus obtained were placed in a test tube. 100 g oftetrafluoro-1,2-diiodoethane (m=1) obtained according to Example 1 were placed in a second test tube. Both open test tubes were put together in a chamber which was then closed. The tetrafluoro-1,2-diiodoethane was then free to expand inside said chamber at room temperature. After 6 days, the test tube containing the solid was extracted from the chamber. Such a solid was washed with $CCl_4$ and dried under reduced pressure. Through an X ray powder diffraction analysis performed on the solid thus obtained, the complete substitution of octafluoro-1,4-diiodobutane (m=2) with tetrafluoro-1,2-diiodoethane (m=1) was noticed, i.e. the formation of a pure adduct of octamethonium iodide (n=8) and tetrafluoro-1,2-diiodoethane (m=1) (in 1:1 ratio) was noticed. Furthermore, the adduct thus formed, i.e. obtained from a gas/solid exchange reaction, had the same crystalline phase of the adduct obtained from solution according to Example 1.

Such a result confirmed the selectivity of the sequestering agent, which, selected according to the relation n=2m+6 of the invention, forms an extremely stable adduct with the α,ω-diiodoperfluorinated compound to be purified having a specific value of m. Such a selectivity is even more clear when considering that, even if crystallised in an adduct with an α,ω-diiodoperfluorinated compound having a value of m close to that to be purified, the α,ω-diiodoperfluorinated compound is anyhow quantitatively sequestered from the sequestering agent having n=2m+6.

The same results also show that the adducts of the present invention can be obtained both in liquid phase, i.e. from a solution containing the impure α,ω-diiodoperfluorinated compound and the sequestering agent, and from gaseous phase on solid phase, i.e. from the impure α,ω-diiodoperfluorinated compound provided in gaseous phase on a solid sequestering agent matrix. In this connection, in order to further show the effectiveness of the process of the invention also in gas/solid reactions, the following examples are reported.

**Example 9**

**Purification of tetrafluoro-1,2-diiodoethane (m=1) (via gas/solid)**

20 mg of octamethonium iodide and 100 mg of tetrafluoro-1,2-diiodoethane of 98% purity (sold by Apollo Scientific Ltd) were put into two different open test tubes. Both said open test tubes were promptly put into a chamber, which was immediately closed. The tetrafluoro-1,2-diiodoethane, being very volatile, expanded inside the closed chamber, thus contacting the solid octamethonium iodide. Once the tetrafluoro-1,2-diiodoethane was left to expand for 6 hours at room temperature, the formation of a yellow solid was observed which was extracted from the chamber, washed with $CCl_4$ and dried under vacuum. On the solid thus obtained, $^1H$ and $^{19}F$ NMR tests were carried out in the presence

of $(CF_3CH_2O)_2$ as internal standard; such tests confirmed the formation of an adduct composed of two compounds in 1:1 ratio. An XRD test was performed on the adduct crystal which confirmed the exclusive presence of octamethonium iodide and tetrafluoro-1,2-diiodoethane and allowed to observe the same crystalline phase of the adduct obtained in Example 1.

The tetrafluoro-1,2-diiodoethane was then separated from the sequestering agent by sublimation from the crystalline adduct under vacuum at a temperature of about 30˚C and recovered by recondensation at a temperature of about -198˚C.

It was then possible to further purify the tetrafluoro-1,2-diiodoethane compound available on the market with a purity of 98% to tetrafluoro-1,2-diiodoethane with a 100% purity, through a process of the invention from gas phase on solid phase, thus obtaining the same optimal results of the process of the invention from liquid phase.

**Example 10**

**Purification of octafluoro-1,4-dilodobutane (m=2) (via gas/solid)**

20 mg of decamethonium iodide and 100 mg of octafluoro-1,4-diiodobutane of 98% purity (sold by Sigma-Aldrich) were put into two different open test tubes. Both said open test tubes were promptly put into a chamber, which was immediately closed. The octafluoro-1,4-diiodobutane, being very volatile, expand inside the closed chamber, thus contacting the solid decamethonium iodide. Once the octafluoro-1,4-diiodobutane was left to spread for 6 hours at room temperature, the formation of a yellow solid was observed which was extracted from the chamber, washed with $CCl_4$ and dried under vacuum. On the solid thus obtained, $^1H$ and $^{19}F$ NMR tests were carried out in the presence of $(CF_3CH_2O)_2$ as internal standard; such tests confirmed the formation of an adduct composed of two compounds in 1:1 ratio. An XRD test was performed on the adduct crystal which confirmed the exclusive presence of decamethonium iodide and octafluoro-1,4-diiodobutane and allowed to observe the same crystalline phase of the adduct obtained in Example 2.

The octafluoro-1,4-diiodobutane was then separated from the sequestering agent by sublimation from the crystalline adduct under vacuum at temperature of about 40˚C and recovered by recondensation at a temperature of about -198˚C.

It was then possible to further purify the octafluoro-1,4-diiodobutane compound available on the market with a 98% purity to octafluoro-1,4-diiodobutane with 100% purity, through a process of the invention from gas phase on solid phase, thus obtaining the same optimal results of the process of the invention from liquid phase.

**Example 11**

**Purification of dodecafluoro-1,6-diiodohexane (m=3) (via gas/solid)**

20 mg of dodecamethonium iodide and 100 mg of dodecafluoro-1,6-diiodohexane of 98% purity (sold by Sigma-Aldrich) were put into two different open test tubes. Both said open test tubes were promptly put into a chamber, which was immediately closed. The dodecafluoro-1,6-diiodohexane, being very volatile, expand inside the closed chamber, thus contacting the solid dodecamethonium iodide. Once the dodecafluoro-1,6-diiodohexane was left to spread for 6 hours at room temperature, the formation of a yellow solid was observed which was extracted from the chamber, washed with $CCl_4$ and dried under vacuum. On the solid thus obtained, $^1H$ and $^{19}F$ NMR tests were carried out in the presence of $(CF_3CH_2O)_2$ as internal standard; such tests confirmed the formation of an adduct composed of two compounds in 1:1 ratio. An XRD test was performed on the adduct crystal which confirmed the exclusive presence of dodecamethonium iodide and dodecafluoro-1,6-diiodohexane and allowed to observe the same crystalline phase of the adduct obtained in Example 3.

The dodecafluoro-1,6-diiodohexane was then separated from the sequestering agent by sublimation of the crystalline adduct under vacuum at a temperature of about 50˚C and recovered by recondensation at a temperature of about -198˚C.

It was then possible to further purify the dodecafluoro-1,6-diiodohexane available on the market with a 98% purity to dodecafluoro-1,6-diiodohexane with a 100% purity, through a process of the invention from gas phase on solid phase, thus obtaining the same optimal results of the process of the invention from liquid phase.

Therefore, the process of the present invention allows to selectively and quantitatively sequester the $\alpha,\omega$-diiodoperfluorinated compound of interest and purify the same, while separating it from the sequestering agent so as to obtain, in significantly high yields, a 100% pure $\alpha,\omega$-diiodoperfluorinated compound. Particularly, it was possible to provide pure, even 100% pure, $\alpha,\omega$-diiodoperfluorinated compounds having also long-chain, i.e. having a number of carbon atoms greater than 8.

Moreover, such a process, unlike the known processes involving fractional distillation, has resulted to be

extremely advantageous from the point of view of implementation and production advantage, through simple and economical steps, since the adduct precipitates and separates at room temperature and the sequestering agent can be reused.

## Claims

1. A process for purifying a α,ω-diiodoperfluorinated compound of formula

$$I\text{-}(CF_2CF_2)_m\text{- }I$$

comprising the steps of:

a) providing **an α,ω-diiodoperfluorinated compound having a purity of less than 95%,**
b) contacting the α,ω-diiodoperfluorinated compound of step a) with a sequestering agent of formula

$$[(CH_3)_3N\text{-}(CH_2)_n\text{-}N(CH_3)_3]^{2+}\cdot 2I^-$$

thus forming an adduct **with the sequestering agent**; and
c) separating the α,ω-diiodoperfluorinated compound from the adduct of step b),
wherein
m is an integer from 1 to 10
and n=2m+6.

2. The process according to claim 1, wherein the sequestering agent is in an amount equimolar to the α,ω-diiodoperfluorinated compound.

3. The process according to claim 1 or 2, wherein step c) of separating the α,ω-diiodoperfluorinated compound from the adduct of step b) is carried out by sublimation of the α,ω-diiodoperfluorinated compound, by means of vacuum application, and subsequent recovery of the same by low temperature recondensation.

4. An adduct of an α,ω-diiodoperfluorinated compound of formula $I\text{-}(CF_2CF_2)_m\text{-}I$ and a sequestering agent of formula

$$[(CH_3)_3N\text{-}(CH_2)_n\text{-}N(CH_3)_3]^{2+}\cdot 2I^-$$

wherein
m is an integer from 1 to 10,
and n=2m+6.

5. Use of an adduct of an α,ω-diiodoperfluorinated compound of formula $I\text{-}(CF_2CF_2)_m\text{-}I$ and a sequestering agent of formula

$$[(CH_3)_3N\text{-}(CH_2)_n\text{-}N(CH_3)_3]^{2+}\cdot 2I^-$$

wherein
m is an integer from 1 to 10
and n=2m+6 for providing α,ω**-diiodoperfluorinated compounds of formula $I\text{-}(CF_2CF_2)_m\text{-}I$ having a purity of at least 95%.**

## Patentansprüche

1. Verfahren zur Aufreinigung einer α,ω-Diiod-perfluorierten Verbindung der Formel

$$I\text{-}(CF_2CF_2)_m\text{-}I$$

umfassend die Schritte des:

a) Bereitstellens einer α,ω-Diiod-perfluorierten Verbindung mit einer Reinheit von weniger als 95%,
b) In-Kontakt-Bringens der α,ω-Diiod-perfluorierten Verbindung aus Schritt a) mit einem Komplexbildner der Formel

$$[(CH_3)_3N\text{-}(CH_2)_n\text{-}N(CH_3)_3]^{2+}\cdot 2I^-$$

wodurch sie mit dem Komplexbildner ein Addukt bildet, und
c) Trennens der α,ω-Diiod-perfluorierten Verbindung vom Addukt aus Schritt b), wobei
m eine ganze Zahl von 1 bis 10 ist
und n = 2m + 6.

**2.** Verfahren nach Anspruch 1, wobei der Komplexbildner in einer Menge vorliegt, die äquimolar zur α,ω-Diiod-perfluorierten Verbindung ist.

**3.** Verfahren nach Anspruch 1 oder 2, wobei Schritt c) des Trennens der α,ω-Diiod-perfluorierten Verbindung vom Addukt aus Schritt b) durch Sublimation der α,ω-Diiod-perfluorierten Verbindung mittels Unterdruck-Anwendung und anschließendes Auffangen derselben durch Rekondensation bei niedriger Temperatur durchgeführt wird.

**4.** Addukt aus einer α,ω-Diiod-perfluorierten Verbindung mit Formel $I\text{-}(CF_2CF_2)_m\text{-}I$ und einem Komplexbildner der Formel

$$[(CH_3)_3N\text{-}(CH_2)_n\text{-}N(CH_3)_3]^{2+}\cdot 2I^-$$

wobei
m eine ganze Zahl von 1 bis 10 ist
und n = 2m + 6.

**5.** Verwendung eines Addukts aus einer α,ω-Diiod-perfluorierten Verbindung mit der Formel $I\text{-}(CF_2CF_2)_m\text{-}I$ und einem Komplexbildner der Formel

$$[(CH_3)_3N\text{-}(CH_2)_n\text{-}N(CH_3)_3]^{2+}\cdot 2I^-$$

wobei
m eine ganze Zahl von 1 bis 10 ist
und n = 2m + 6, um α,ω-Diiod-perfluorierte Verbindungen der Formel $I\text{-}(CF_2CF_2)_m\text{-}I$ mit einer Reinheit von mindestens 95% bereitzustellen.

## Revendications

**1.** Procédé de purification d'un composé α,ω-diiodoperfluoré de formule

$$I\text{-}(CF_2CF_2)_m\text{-}I$$

comprenant les étapes consistant à:

a) fournir un composé α,ω-diiodoperfluoré ayant une pureté inférieure à 95 %,
b) mettre en contact le composé α,ω-diiodoperfluoré de l'étape a) avec un agent séquestrant de formule

$$[(CH_3)_3N\text{-}(CH_2)_n\text{-}N(CH_3)_3]^{2+}\cdot 2I^-$$

formant ainsi un adduit avec ledit agent séquestrant, et
c) séparer le composé α,ω-diiodoperfluoré de l'adduit de l'étape b), dans lesquelles
m est un nombre entier de 1 à 10 et
n=2m+6.

**2.** Procédé selon la revendication 1, dans lequel ledit agent séquestrant est présent en une quantité équimolaire au composé α,ω-diiodoperfluoré.

3. Procédé selon la revendication 1 ou 2, dans lequel ladite étape c) consistant à séparer le composé α,ω-diiodoperfluoré de l'adduit de l'étape b) est mise en oeuvre par sublimation du composé α,ω-diiodoperfluoré, au moyen d'une application du vide, et par récupération subséquente de celui-ci par une recondensation à faible température.

4. Adduit d'un composé α,ω-diiodoperfluoré de formule $I\text{-}(CF_2CF_2)_m\text{-}I$ et d'un agent séquestrant de formule

$$[(CH_3)_3N\text{-}(CH_2)_n\text{-}N(CH_3)_3]^{2+}\cdot 2I^-$$

dans lesquelles
m est un nombre entier de 1 à 10 et
n=2m+6.

5. Utilisation d'un adduit d'un composé α,ω-diiodoperfluoré de formule $I\text{-}(CF_2CF_2)_m\text{-}I$ et d'un agent séquestrant de formule

$$[(CH_3)_3N\text{-}(CH_2)_n\text{-}N(CH_3)_3]^{2+}\cdot 2I^-$$

dans lesquelles
m est un nombre entier de 1 à 10 et
n = 2 m + 6, afin de fournir des composés α,ω-diiodoperfluorés de formule $I\text{-}(CF_2CF_2)_m\text{-}I$ ayant une pureté d'au moins 95 %.

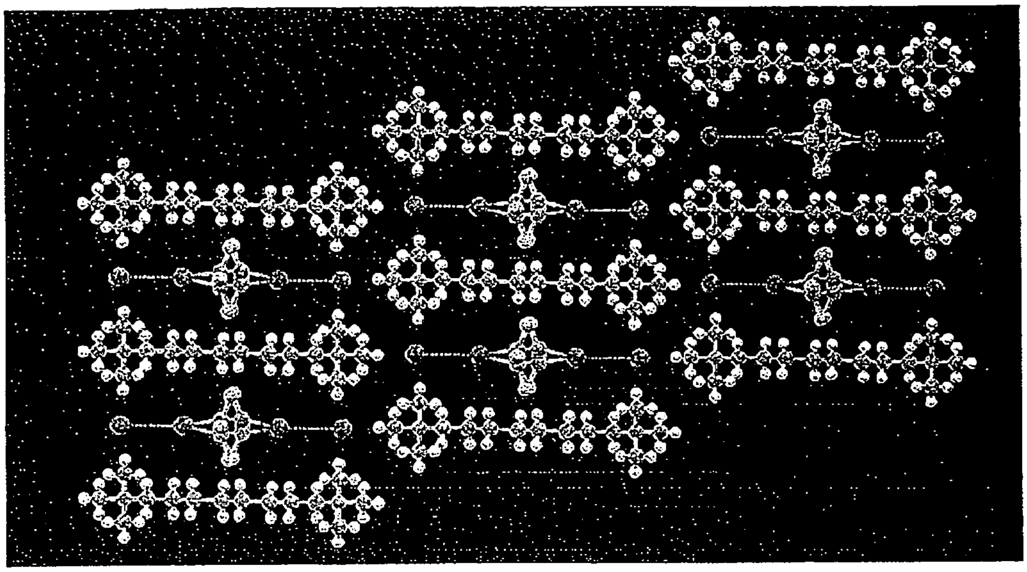

Figure 1

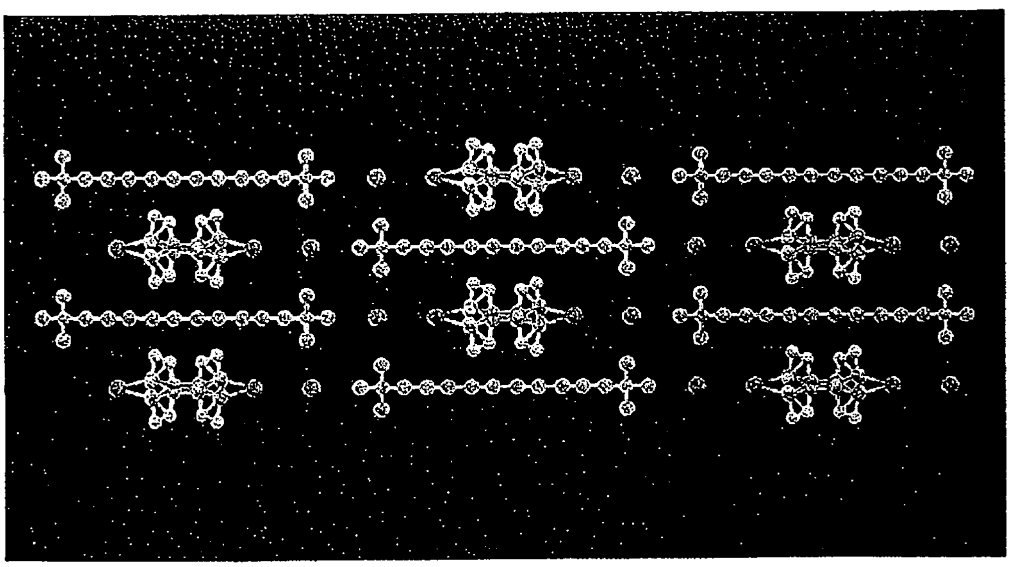

Figure 2

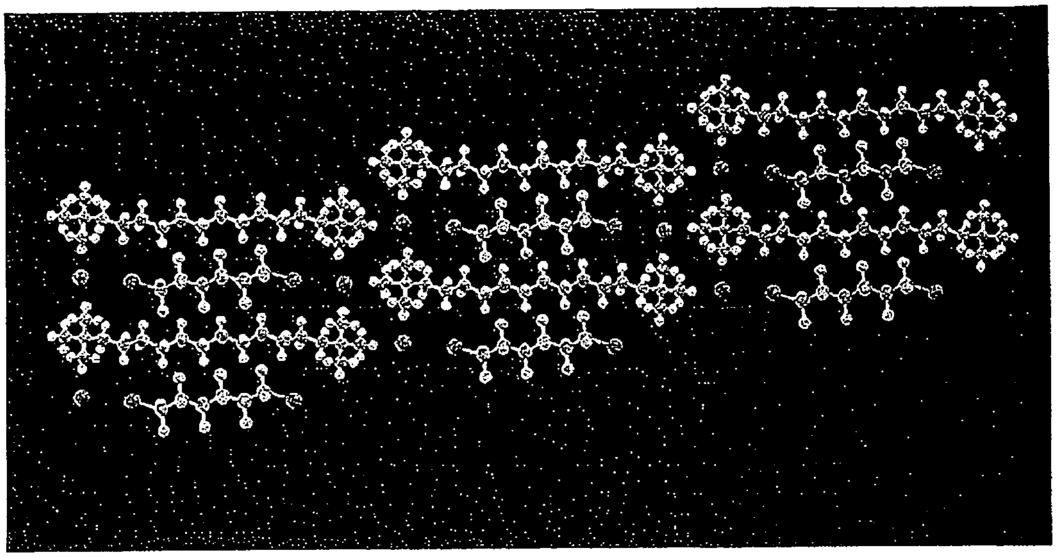

Figure 3

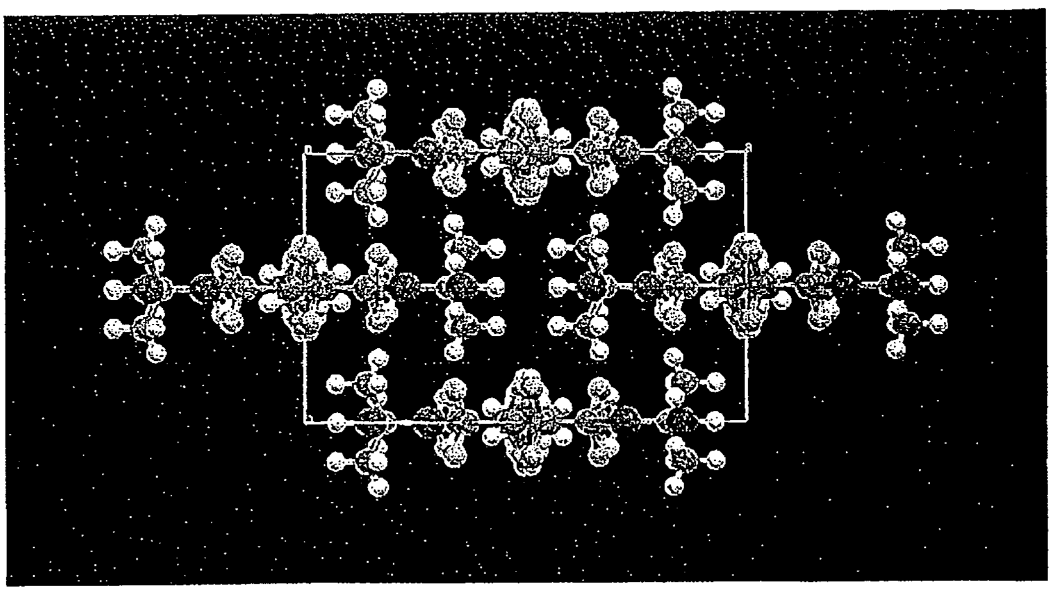

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5214106 A
- US 6150565 A
- US 6002055 A
- US 4731170 A
- US 6825389 B


### Non-patent literature cited in the description


- **Fox BF et al.** *Journal of Fluorine Chemistry,* 2004, vol. 125, 271-281
- **Gattuso G et al.** *Tetrahedron,* 2007, vol. 63, 4951-4958
- **Casnati A et al.** *Angew. Chem. Int.,* 2006, vol. 45, 1915-1918
- **Nair et al.** *Journal of Fluorine Chemistry,* 1993, vol. 60, 1-12
- **J. Zhang et al.** *J Mol. Struct.,* 2003, vol. 660, 119-129
- **Dang T.A. et al.** *Analytical chemistry,* May 1984, vol. 56 (6), 866-871
- **Lee C et al.** *Brit. J. Anaesthesia, sol.,* 2001, vol. 88 (5), 692-699
- The synthesis of methonium compounds, their isolation from urine, and their photometric determination. **Zaimis, Eleanor J.** British Journal of Pharmacology and Chemotherapy. Natl. Inst. Med. Research, London, 1950, vol. 5, 424-30
- **Fiori A. et al.** *Journal of Chomatography,* 1967, vol. 31, 171-176